# DEMANDE DE BREVET EUROPEEN

(11) **EP 4 378 429 A1**
(43) Date de publication de la demande: **05.06.2024**
(21) Numéro de dépôt: 22020589.2
(22) Date de dépôt: 01.12.2022
(51) Int. Cl.: A61F 2/58, B25J 15/00

(54) **PROTHÈSE DE MAIN MOTORISÉE MULTIARTICULÉE**

(71) Demandeur: Feltrin, Oscar, 47825 Poggio Torriana (RN) (IT)
(72) Inventeur: Feltrin, Oscar, 47825 Poggio Torriana (RN) (IT)

(57) **Abrégé**

Prothèse de main à fonctionnement éléctrique comprenant des doigts longs poliarticulés (II,III,IV,V) mis en mouvement de fléxion ou en extension par un seul axe de rotation (F) par l'intermédiaire des câbles (L1,L2,L3) pour la fléxion situé côté palmaire, et des câbles pour l'extension (L4,L5,L6) coté dorsal, ces doigts ont une adaptation mécanique à l'objet à tenir, cette main comporte aussi un pouce à ouverture et fermeture éléctrique avec position latérale manuelle.

## Description

La présente invention concerne une prothèse de main antropomorphe multi-articulée à adaptation mécanique des phalanges à l'objet à saisir, à fonctionnement éléctrique comprenant 4 doigts articulés mis en mouvement de fléxion ou d'extension sans resort de rappel, par un seul moteur et un seul axe de rotation muni d'une roue hélicoïdale et de deux poulies de tirage par l'intermédiaire de fléaux et de câbles de répartition des forces pour la fléxion et l'extension et d'un pouce motorisé indépendant. La répartition en fléxion et en extension des forces sur les doigts longs se fait par câbles fixés aux fléaux de tirage des doigts longs, chaque fléau est muni de deux câbles en U pour la traction ou la fléxion des doigts longs. Cette répatition des forces permet à ces doigts une adaptation mécanique à l'objet à tenir comme il sera expliqué plus avant. Même si actuellement il existe déjà des prothéses poliarticulées, celles-ci ont généralement plus de deux moteurs et qui de ce fait sont d'un poids non indifférent qui souvent est une gêne pour l'utilisateur.

Dans la littérature de la chirurgie de la main, le pouce est consideré comme 50% de la fonctionnalité de celle-ci, c'est pourquoi ce dispositif est muni d'un pouce éléctrique indépendant.

Dans le domaine de la prothèse il existe des prothèses de main comme par exemple le brevet FR 2957245 qui prévoit un moteur central et un tirage par ruban passant entre 4 roues percées et le pouce n'est pas motorisé, le brevet US2549716 qui consiste en tirages avec des barres rigides et avec rappel à ressort, toujours à compenser par une force antagoniste, le brevet FR 942172 qui comporte des tirages complexes par poulies et un pouce fixe positionnable manuellement, le brevet EP 2890333 qui consiste en 2 moteurs centralisés qui tirent chacun deux poulies et un moteur de pouce fonctionnant à engrenages latéraux, le brevet US2006/129248 où la fléxion des doigts se fait uniquement par ressort, le brevet Europeen EP2890333 qui comporte 3 moteurs et 3 tirages différents pour chaque moteur, le brevet WO2019115665 qui est une main à fonctionnement cinématique pour pince tridigitale, le brevet WO2019215577 dont la répartition des forces est exercée à moitié sur les doigts IV et V et l'autre moitié sur les 3 doigts restants avec une aide au retour par éléments élastiques, le brevet WO2019116321 qui concerne une main mue par 5 moteurs indépendants tout comme le brevet EP2542189, le brevet WO2016005871 qui concerne une main mue en fléxion par 5 fléaux interconnectés et extension comme la main du brevet FR2665833, le brevet WO2007076795 qui concerne une prothése à pince tridigitale sans articulations phalangées comme le brevet EP1962733, le brevet WO2017111582 à fonctionnement par ressorts et par paire de doigts non indépendants, le brevet 2010018358 qui comporte 4 moteurs pour son fonctionnement, de tous les brevets cités aucun ne prévoit le mouvement des 4 doigts avec un seul axe en rotation, un seul fléau pour l'extension des 4 doigts longs et un pour la flexion de ces 4 doigts et sans moyens de rappel élastique pour ces 5 doigts.

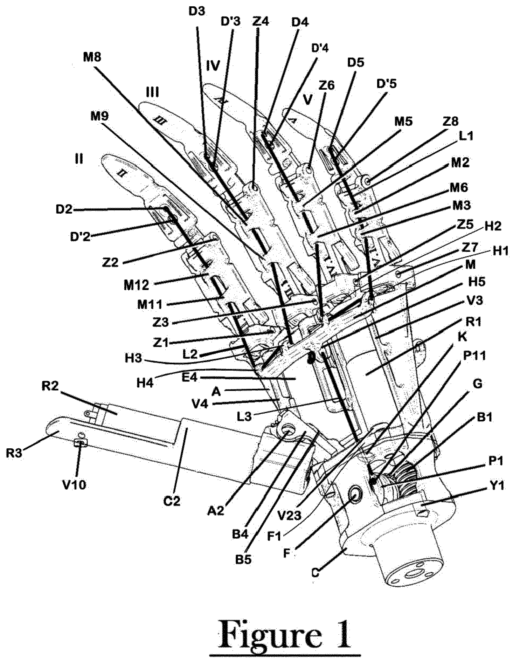

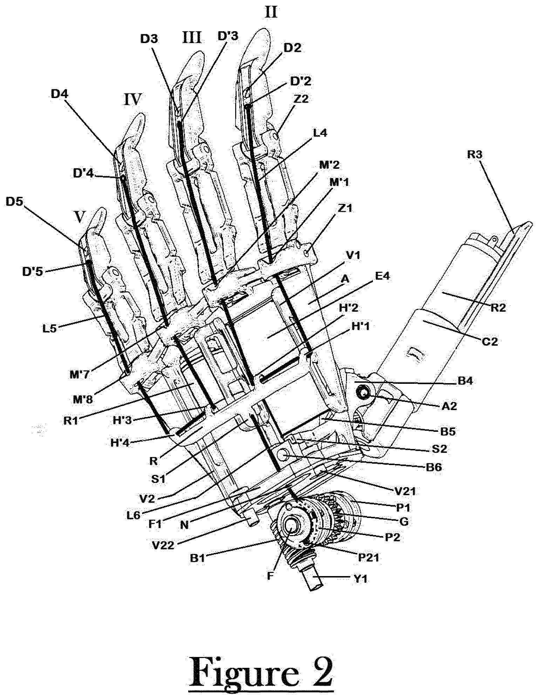

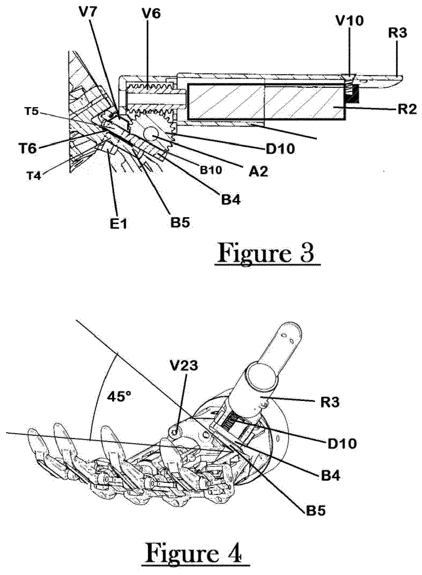

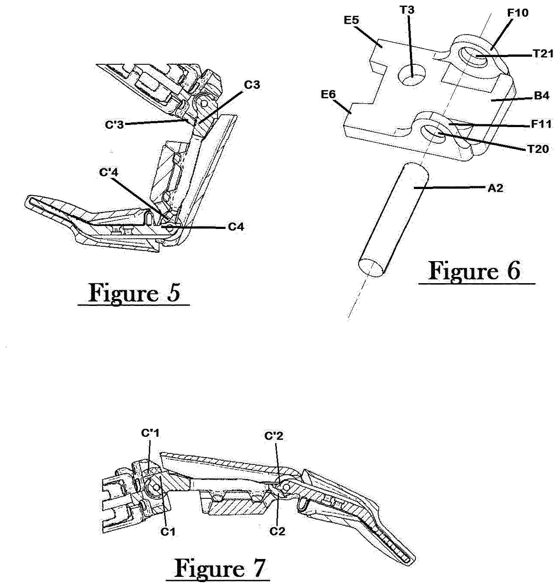

La présente invention a pour but de simplifier et remédier aux inconvénients des brevets précités en décrivant une prothése de main dont la fléxion et l'extension des 4 doigts longs articulés peut être réalisée à l'aide d'un seul moteur, d'un seul axe de rotation et un seul fléau pour la fléxion des 4 doigts longs et un seul fléau pour l'extension de ces 4 doigts longs et un retour en extension sans moyens de rappel pour les 4 doigts, le but étant de ne pas utiliser de ressorts de rappel pour quelque fonction que ce soit pour toujours profiter du maximum de puissance du moteur et un pouce à fonctionnement éléctrique indépendant. Le pouce est articulé sur l'axe A2 [Fig. 1], [Fig.2], [Fig.3], [Fig.6] et le mouvement latéral se fait manuellement autour de l'axe V7 [Fig.3], les éléctrodes et les batteries sont situées de maniére classique dans l'avant-bras prothétique, seuls les fils d'alimentation arrivent dans la prothèse de main, l'éléctronique E4 [Fig.1], [Fig.2], distribue les fonctions au moteur des doigts et à celui du pouce, la séléction des programmes se fait avec le bouton poussoir B6 [Fig.2].

Cette prothèse de main à fonctionnement éléctrique comprend :
une base A comportant le support des axes Z1, Z3, Z5, Z7 [Fig.1] des doigts prothétiques, une base B5 [Fig.1], [Fig.2], [Fig.3] ayant une inclination de 45 degrés par rapport au plat de la base A [Fig.1], [Fig.2], [Fig.4] qui supporte le pouce R3 et un trou T6 [Fig.3] destiné à recevoir la bride métallique B4 [Fig.1], [Fig.2], [Fig.3], [Fig.4], [Fig.6] du pouce munie de deux languettes F10 et F11 munies de deux trous T20, T21 [Fig.6] servant de support à l'axe A2 et qui pivote sur la vis V7 [Fig.3], la plaque F1 [Fig. 1], [Fig.2] qui fait partie de la base A, est percée pour la fixation de la cage C [Fig.1] à l'aide des vis V21,V22 [Fig.2] et V23 [Fig.1] et la fixation du moteur R1 [Fig.1], [Fig.2] et des trous K [Fig.1] et N [Fig.2] pour le passage des câbles de tirage, un support de bouton poussoir S2 [Fig.2], un emplacement destiné à recevoir l'éléctronique E4 [Fig.1], [Fig.2], quatre doigts longs II,III,IV,V prothétiques qui sont articulés respectivement pour le doigt II sur les axes Z1 et Z2 [Fig.1], [Fig.2], le doigt III sur les axes Z3, Z4 [Fig.1], le doigt IV sur les axes Z5, Z6 [Fig.1], le doigt V sur les axes Z7, Z8 [Fig.1], le doigt II comprend des passants M12,M11 côté palmaire et des trous de fixation D2 et D'2 [Fig.1], [Fig.2] sur la troisième phalange, le doigt III comprend des passants M8,M9 côté palmaire et des trous de fixation D3 et D'3 [Fig.1], [Fig.2] sur la troisième phalange, le doigt IV comprenant les passants M5, M6 côté palmaire et des trous de fixation D4 et D'4 [Fig.1], [Fig.2] sur la troisième phalange, le doigt V comprenant les passants M2,M3 côté palmaire et des trous de fixation D5 et D'5 [Fig1, [Fig.2] sur la troisième phalange, un pouce R3 articulé sur l'axe A2 [Fig.1], [Fig.2], [Fig.3], et articulé latéralement sur l'axe V7 [Fig.3], ce pouce est formé d'une cage C2 [Fig.1], [Fig.2], [Fig.3], comprenant à son intérieur un moteur R2 [Fig.1], [Fig.2], [Fig.3] muni à son axe de rotation d'une vis sans fin V6 [Fig.3] couplée à la roue hélicoïdale D10 par l'intermédiaire de l'axe de rotation A2 [Fig.3], la roue hélicoïdale ayant un méplat B10 [Fig.3] est donc fixe sur sa bride métallique B4 [Fig.3], [Fig.6] munie des deux languettes F10 et F11 [Fig.6] munies de deux trous T20, T21 servant de support à l'axe A2 [Fig.6] portant la roue hélicoïdale D10 [Fig.3] et de deux ergots E5 et E6 [Fig.6] permettant de limiter les déplacements latéraux du pouce, une vis V10 [Fig.3] de blocage du moteur R2 [Fig.3], l'ensemble à vis sans fin et roue hélicoïdale du pouce forme un dispositif de blocage irréversible quand le moteur ne fonctionne pas. Une cage C [Fig.1] servant respectivement de support au moteur R1 [Fig.1] [Fig.2], à la vis sans fin B1 et à son l'axe de rotation Y1 [Fig.1] [Fig.2], de support à un axe de rotation F solidaire sur sa partie centrale de la roue hélicoïdale G [Fig.1], [Fig.2] qui est entraînée par la vis sans fin (B1). De chaque côté de la roue hélicoïdale G [Fig.1], [Fig.2] sur l'axe F sont fixées les poulies P1 et P2 [Fig.2] qui servent de tirage en fléxion et en extension les doigts longs, la poulie P2 [Fig2] est munie d'un axe P21 qui sert de tirage au câble L6 [Fig.2], la poulie P1 [Fig.1] est munie d'un axe P11 qui sert de tirage au câble L3. Un fléau dorsal R comprend des passants H'1, H'2, H'3, H'4 [Fig.2] ou passent les câbles L4, L5 et un anneau d'ancrage S1 où est fixé le câble L6 [Fig.2] qui servent de tirage en extension aux doigts longs, un fléau palmaire M comprenant les passants H1, H2, H3, H4 [Fig.1] où passent les câbles L1, L2 et un anneau d'ancrage H5 où est fixé le câble, L3 [Fig.1] qui servent de tirage en fléxion aux doigts longs.

Description des dessins:
[Fig. 1] : vue palmaire main ouverte avec moteurs et circuit éléctronique
[Fig. 2] : vue dorsale main ouverte avec moteurs et sans la cage C
[Fig. 3] : vue en coupe du pouce
[Fig. 4] : vue de la main à plat
[Fig. 5] : vue en coupe avec doigt en fléxion
[Fig. 6] : vue du support du pouce
[Fig. 7] : vue en coupe avec doigt en extension

Prothèse de main comprenant une base de main A [Fig.1], [Fig.2], [Fig.4] sur laquelle sont fixés les 4 doigts longs, II, III, IV V, le pouce éléctrique, les fléaux, le dispositif de déplacement manuel du pouce, le circuit éléctronique E4 de commande et un bouton poussoir B6 de selection de programme, le poignet comprenant la cage C où est fixé le moteur, le dispositif de tirage en fléxion et en extension, les câbles.

Selon une forme de réalisation, la base de la main A [Fig1], [Fig.2], [Fig.4] sert de support au pouce R3 sur la base B5 [Fig.1], [Fig.2], [Fig.3] et sert de support au moteur R1 [Fig.1], [Fig.2], qui sur son axe de rotation Y1 est fixée une vis sans fin B1 [Fig1], [Fig.2] qui est accouplée à la roue hélicoïdale G [Fig.2]. La cage C [Fig.1] qui est fixée sur la base de la main A par la plaque F1 [Fig.1], [Fig.2] à l'aide des vis V21,V22,V23 [Fig.1], [Fig.2] sert respectivement de support à la vis sans fin B1 et à l'axe de rotation F [Fig.1], [Fig.2] et au moteur R1. Sur cet axe de rotation F est fixée la poulie P1 [Fig.1], [Fig.2] qui sert de tirage en fléxion aux quatre doigts longs et la poulie P2 [Fig.2] qui sert de tirage en extension des quatre doigts longs, et entre ces deux poulies est fixée la roue hélicoïdale G [Fig.1], [Fig.2] qui sert à entraîner l'axe F, les deux poulies et la roue hélicoïdale G sont solidaires de l'axe F [Fig.1], [Fig.2]. Les poulies P1 [Fig.1] et P2 [Fig.2] sont munies respéctivement d'un axe P11 et P21 permettant la fixation des câbles d'extension L6 [Fig.2], et de fléxion L3 [Fig.1] et d'exercer les tirages sur les fléaux M [Fig.1], et R [Fig.2] qui eux permettent de répartir les forces exercées sur les quatre doigts longs. Le demi-tour que fait la roue hélicoïdale G [Fig.1], [Fig.2] est calculé pour qu'aux poulies P1 et P2 corresponde un tirage d'une longueur de câble nécessaire et suffisante pour permettre l'ouverture ou la fermeture complète des doigts, il est à noter que si même la roue hélicoïdale G devait faire plus d'un demi-tour, il suffira de prévoir de décaler l'axe de tirage opposé à une des deux poulies. Selon une autre forme avantageuse de la réalisation, le dispositif de fonctionnement des doigts en fléxion est formé d'un fléau M [Fig.1] avec des passants H1, H2, H3, H4 [Fig.1], permettant le passage des câbles de fléxion L1,L2 et l'anneau d'ancrage H5 pour le câble L3 [Fig.1], le fonctionnement est le suivant du côté palmaire :
Le fléau M est muni d'un passant d'ancrage H5 [Fig.1] d'un côté et de l'autre de quatre passants H1, H2, H3, H4 [Fig.1]. Un câble de tirage L1 [Fig.1] est fixé à l'extrémité du doigt V dans le trou d'ancrage D5 [Fig.1] avec un noeud côté dorsal, passe dans les passants M2, M3, par les passants H1 et H2, passe dans les passants M6,M5 [Fig.1] et se fixe à l'extrémité du doigt IV au trou d'ancrage D4 [Fig.1] côté dorsal par un noeud, il en va de même pour le câble L2 [Fig.1] qui est fixé à l'extrémité du doigt III au trou d'ancrage D3 côté dorsal à l'aide d'un noeud, passe dans les passants M8, M9 [Fig.1] dans les passants H3 et H4, passe dans les passants M11,M12, puis va se fixer à l'extrémité du doigt II au trou d'ancrage D2 [Fig.1] côté dorsal par un nœud, en tirant sur ce fléau M [Fig.1] comportant deux câbles en forme de U, un équilibre se crée et permet une adaptation souple aux objets à saisir par la main, le tirage de ce fléau M se fait par la poulie P1 [Fig.1], le câble L3 est fixé à une extrêmité sur l'anneau d'ancrage H5 [Fig.1] du fléau, passe dans le trou passant K de la flange F1 [Fig.1], et va se fixer autour de l'axe P11 de la poulie P1 [Fig.1]. Quand l'axe P11 tire sur le câble L3 [Fig.1], les câbles L1 et L2 [Fig.1] coulissent sur leurs passants respectifs du fléau M et permettent à ces doigts de s'adapter à l'objet à saisir. On peut remarquer que quand le câble de tirage en fléxion L3 est en traction, le câble d'extension L6 se relâche. Le blocage des doigts longs en mode prise d'un objet, s'effectue par la roue hélicoïdale G avec la vis sans fin B1 [Fig.1] et [Fig.2] qui est un dispositif irréversible quand le moteur ne fonctionne pas. Selon une autre forme avantageuse de la réalisation, le dispositif de fonctionnement des doigts en extension est formé d'un fléau R [Fig.2] avec des passants H'1,H'2,H'3,H'4 permettant le passage des câbles L4 et L5 en extension et un anneau d'ancrage S1 pour le câble L6, le fonctionnement est le suivant du côté dorsal :
Le fléau R [Fig.2] est muni d'un passant d'ancrage S1 d'un côté et de l'autre côté de quatre passants H'1, H'2, H'3, H'4 [Fig.2]. Un câble de tirage L4 est fixé à l'extrêmité du doigt II dans le trou d'ancrage D'2 côté palmaire par un noeud, passe dans les passants M'1, H'1, H'2, M'2 [Fig.2] et se fixe à l'extrêmité du doigt III au trou d'ancrage D'3 côté palmaire par un noeud, il en va de même pour le câble L5 [Fig.2] qui est fixé à l'extrêmité du doigt IV au trou d'ancrage D'4 coté palmaire à l'aide d'un noeud, passe dans les passants M'7, H'3, H'4, M'8, [Fig.2], puis va se fixer à l'extrêmité du doigt V au trou d'ancrage D'5 côté palmaire par un noeud, en tirant sur ce fléau un équilibre se crée et permet l'extension des doigts longs. Le tirage sur ce fléau R se fait par la poulie P2 [Fig.2] et le câble L6 qui est fixé à une extrêmité sur l'anneau d'ancrage S1 [Fig.2] du fléau R, passe dans le trou passant N [Fig.2] de la plaque F1, et va se fixer autour de l'axe P21 de la poulie P2 [Fig.2], Quand l'axe P21 tire sur le câble L6, les câbles L4 et L5 coulissent sur leurs passants respectifs du fléau créant un équilibre de forces et permettant aux doigts de se mettre en extension.

Selon une autre forme avantageuse de la réalisation, le pouce est formé d'une bride métallique B4 [Fig.1], [Fig.2], [Fig.3] et d'une cage C2 [Fig.1], [Fig.2], [Fig.3] comprenant à son intérieur un moteur R2 muni à son axe d'une vis sans fin V6 couplée à la roue hélicoïdale D10 [Fig.3] par l'intermédiaire de l'axe de rotation A2 [Fig.3], la roue hélicoïdale D10 ayant un méplat B10 est donc fixe sur sa bride métallique B4 [Fig.1], [Fig.2], [Fig.3], [Fig.4] [Fig.6] munie de deux languettes F10 et F11 ayant deux trous T20 et T21 [Fig.6] servant de support à l'axe A2 portant la roue hélicoïdale D10 [Fig.3], et de deux ergots E5 et E6 [Fig.6] permettant de limiter les déplacements latéraux du pouce, l'ensemble à vis sans fin et roue hélicoïdale forme un dispositif de blocage irréversible quand le moteur ne fonctionne pas, et qui lui aussi peut se mettre en opposition aux quatre doigts longs, l'ensemble des ces deux dispositifs permet le blocage de l'objet saisi.

Selon une forme avantageuse de la réalisation, la bride métallique B4 [Fig.6] du pouce peut être mise en rotation sur le support B5 [Fig.3] par l'intermédiaire de la vis V7 [Fig.3] passant dans les trous T5 et T4 [Fig.3], [Fig.6] et de l'écrou auto-bloccant E1, une rondelle en teflon ou nylon T6 [Fig.3] est inserrée entre la bride métallique B4 et son support B5 [Fig.3] et permet une rotation souple entre ces deux matériaux et de pouvoir pivoter latéralement quand on exerce une poussée latérale sur le pouce R3.

Selon un autre avantage de la réalisation, les languettes V1,V2, [Fig.2] du côté dorsal et les languettes V3,V4, du côté palmaire [Fig.1], [Fig.4] sont d'une hauteur nécessaire et suffisante pour protéger le moteur R1 et l'éléctronique E4 [Fig.1], [Fig.2] et permettre le glissement des fléaux R et M [Fig.1], [Fig.2].

Selon une forme avantageuse de la réalisation, chaque doigt long à des butées d'ouverture maximum, C1 sur C'1 et C2 sur C'2 [Fig.7] et des butées de fermeture maximum, C3 sur C'3 et C4 sur C'4 [fig.5], ces butées en fonction de leur dimension limitent la fléxion et/ou l'extension maximum des doigts selon l'amplitude désirée par l'utilisateur.

Selon une forme avantageuse de la réalisation, l'éléctronique E4 [Fig.1], [Fig.2] distribue les fonctions au moteur R1 des doigts longs et au moteur R2 du pouce par un bouton poussoir B6 [Fig.2] qui effleure la partie dorsale de la main permettant de choisir son programme d'utilisation sans que celui-ci soit visible à l'extérieur du gant de recouvrement.

Un capotage de protection de toute la partie palmaire et dorsale est ensuite ajouté pour la protection et l'esthétique de la main.

## Revendications

1. Prothèse de main à fonctionnement éléctrique avec doigts à phalanges multiarticulées et pouce éléctrique indépendant à positionnement latéral manuel, comprenant une base de main (A) sur laquelle sont fixés un moteur (R1) avec sur son axe de rotation (Y1) la vis sans fin (B1), des languettes de protection du moteur et de l'éléctronique (V1,V2,V3,V4), une éléctronique (E4) interne à la main pour la commande des moteurs des doigts longs et du pouce, un support de bouton poussoir et de son bouton poussoir (B6) qui sert de programmation aux deux moteurs (R1,R2) de la main, une cage (C) comprenant un axe de rotation (F), muni d'une roue hélicoïdale (G) solidaire de cet axe (F) ainsi que des deux poulies (P1) et (P2), la poulie (P1) servant de tirage en fléxion aux quatre doigts longs par son axe de tirage (P11) et la poulie (P2) servant d'extension aux quatre doigts longs par son axe de tirage (P21), 4 doigts (II, III, IV, V) à phalanges articulées respectivement par les axes (Z1,Z2) (Z3,Z4) (Z5,Z6) et (Z7,Z8), un pouce éléctrique (R3) formé d'une cage (C2) comprenant à son intérieur un moteur (R2) muni à son axe d'une vis sans fin (V6) formant avec la roue hélicoïdale (D10) et son axe (A2) un dispositif auto-bloccant quand le moteur est à l'arrêt, la roue hélicoïdale comprenant un méplat (B10), elle reste fixe sur sa bride métallique (B4) munie de deux languettes (F10 et F11) percées de deux trous (T20, T21) servant de support à l'axe (A2) de deux ergots (E5) et (E6) permettant de limiter les déplacements latéraux du pouce, un dispositif de déplacement latéral manuel du pouce comprenant une vis (V7), un trou (T5) dans le support (B4), un écrou frein (E1) et une rondelle en téflon ou nylon entre le support (B4) et le support (B5) positionné à 45 degrés par rapport à la base de la main (A), un câble (L6) de tirage du fléau (R) fixé d'un côté à son anneau d'ancrage (S1) et de l'autre côté à l'axe (P21 ), un câble (L3) de tirage du fléau (M) fixé d'un côté à son anneau d'ancrage (H5) et de l'autre côté à l'axe (P11), un fléau (R) comprenant les passants (H'1,H'2,H'3,H'4) pour le tirage en extension des doigts longs et l'anneau d'ancrage (S1), le fléau (M) comprenant les passants (H1,H2,H3,H4) pour le tirage en fléxion des doigts longs et l'anneau d'ancrage (H5), deux câbles (L4,L5) passant respectivement dans les passants (H'1,H'2) et (H'3 et H'4) pour l'extension des doigts longs et deux câbles (L1, L2) passant respectivement par les passants (H1,H2) et (H3,H4) pour la fléxion des doigts longs, une cage (C) fixée à la base de la main (A) sur sa plaque (F1) et servant de support au moteur (R1), à l'axe (Y1) de la vis sans fin (B1), et l' axe de rotation (F) sur lequel sont fixés deux poulies (P1, P2), et une roue hélicoïdale (G) située entre ces deux poulies

2. Prothèse de main articulée à fonctionnement éléctrique selon la revendication 1, **caractérisée par** un dispositif pour la fléxion et l'extension des quatre doigts longs comprenant un seul axe de rotation (F) solidaire de la roue hélicoïdale (G) et de la poulie de tirage (P1) pour le tirage en fléxion des doigts longs et de la poulie (P2) pour le tirage en extension de ces mêmes doigts, la poulie (P1) est munie d'un axe de tirage (P11) et la poulie (P2) est munie d'un axe de tirage (P21), le câble de tirage en fléxion (L3) est fixé d'un côté à l'anneau d'ancrage (H5) du fléau (M), de l'autre à l'axe (P21), le câble de tirage en extension (L6) est fixé d'un côté à l'anneau d'ancrage (S1) du fléau (R), de l'autre à l'axe (P21), en actionnant le moteur (R1) qui fait tourner la vis sans fin(B1) fait exercer une rotation à la roue hélicoïdale (G) et donc des poulies (P1 et P2), il se produit soit un tirage soit un relâchement des câbles (L6) ou (L3) et cela entraîne soit le tirage du fléau (M) soit le tirage du fléau (R), cela permet le mouvement des doigts longs en fléxion ou en extension.

3. Prothèse de main articulée à fonctionnement éléctrique selon la revendication 1, **caractérisée par** un dispositif de répartition des forces comprenant deux fléaux (R ) et (M) servant de tirage en fléxion et en extension les quatre doigts longs, le fléau (R ) de tirage en extension est muni de deux passants (H'1,H'2) qui permettent le coulissement du câble (L4) en forme de U et donc un équilibrage des forces exercées en extension sur les doigts (II) et (III) et de deux passants (H'3,H'4) qui permettent le coulissement du câble (L5) en forme de U et donc un équilibrage des forces exercées en extension sur les doigts (IV) et (V) et d'un anneau d'ancrage (S1) qui sert de tirage au câble (L6), le fléau (M) de tirage en fléxion est muni de deux passants (H1,H2) qui permettent le coulissement du câble (L1) en forme de U et donc un équilibrage des forces exercées en fléxion sur les doigts (IV) et (V) et de deux passants (H3,H4) qui permettent le coulissement du câble (L2) en forme de U et donc un équilibrage des forces exercées en fléxion sur les doigts (II) et (III) et d'un anneau d'ancrage (H5) qui sert de tirage au câble (L3)

4. Prothèse de main articulée à fonctionnement éléctrique selon les revendications 1 et 3 **caractérisée par** un dispositif de tirage en fléxion et extension comprenant une cage (C) qui sert de support au moteur (R1) qui met en rotation la vis sans fin (B1) couplée à la roue hélicoïdale (G), un axe de rotation (F) solidaire de la roue hélicoïdale (G), de la poulie (P1) sert de tirage en fléxion et de la poulie (P2) qui sert de tirage en extension des quatre doigts longs, le demi-tour que fait la roue hélicoïdale (G) est calculé pour qu'aux poulies (P1) et (P2), donc aux axes (P11) et (P22) corresponde un tirage d'une longueur de câble nécessaire et suffisante pour permettre l'ouverture ou la fermeture complète de la main, même si la roue hélicoïdale (G) fait plus d'un demi-tour pour mettre en extension et en fléxion les doigts, il suffira de prévoir de décaler l'axe de tirage (P11) ou (P21) à l'une des deux poulies.

5. Prothèse de main articulée à fonctionnement éléctrique selon la revendication 1 **caractérisée par** un pouce (R3) formé de son support (B4) et d'une cage (C2) comprenant à son intérieur un moteur (R2) muni à son axe d'une vis sans fin (V6) couplée à la roue hélicoïdale (D10) par l'intermédiaire de l'axe de rotation (A2), la roue hélicoïdale ayant un méplat (B10) est donc fixe sur sa bride métallique (B4) munie de deux languettes (F10) et (F11) munies de deux trous (T20, T21) servant de support à l'axe (A2) et portant la roue helicoïdale (D10), de deux ergots (E5) et (E6) permettant de limiter les déplacements latéraux du pouce, l'ensemble à vis sans fin et roue hélicoïdale forme un dispositif de blocage irréversible quand le moteur ne fonctionne pas, et qui lui aussi se met en opposition aux quatre doigts longs, l'ensemble des deux dispositifs des doigts et du pouce permettent le blocage de l'objet saisi.

6. Prothèse de main éléctrique selon la revendication 1 **caractérisée par** le support (B5) du pouce ayant une inclinaison de environ 45 degrés par rapport au support de main (A), la bride métallique (B4) du pouce est fixée sur le support (B5) par l'intermédiaire de la vis (V7) et de l'ecrou auto-bloccant (E1), une rondelle (T6) en teflon ou nylon est inserrée entre la bride métallique (B4) et le support (B5) pour une rotation souple entre ces deux matériaux et permet au pouce de pouvoir pivoter latéralement quand on y exerce une poussée sur celui-ci. La bride métallique (B4) est munie de deux ergots (E5) et (E6) permettant de limiter les déplacements latéraux du pouce qui peut se mettre soit en opposition aux doigts longs soit en position pince latérale.

7. Prothèse de main éléctrique selon la revendication1 **caractérisée par** des languettes (V1 ,V2), du côté dorsal et les languettes (V3,V4), du côté palmaire qui sont d'une hauteur nécessaire et suffisante pour protéger le moteur et l'éléctronique et permettre le glissement des fléaux (R) et (M).

8. Prothèse de main éléctrique selon la revendication 1, **caractérisée en ce que** chaque doigt long a des butées d'ouverture maximum, (C1) sur (C'1) et (C2) sur (C'2) et des butées de fermeture maximum, (C3) sur (C'3) et (C4) sur (C'4), ces butées en fonction de leur dimension limitent la fléxion et l'extension maximum des doigts selon l'amplitude désirée par l'utilisateur.

9. Prothèse de main éléctrique selon la revendication1 **caractérisée par** une éléctronique (E4) interne qui alimente en fonction du programme choisi le et/ou les moteurs (R1) et/ ou (R2) de la main à la main prothétique, cette main est munie d'un bouton poussoir (B6) qui effleure le dorsal de la main permettant de choisir son programme d'utilisation sans que celui-ci soit visible de l'extérieur du gant de recouvrement.

## Revendications modifiées

### Revendications modifiées conformément à la règle 137(2) CBE.

1. Prothèse de main à fonctionnement électrique avec doigts à phalanges multiarticulées et une électronique de commande comprenant:
- une base de main sur laquelle sont fixés le pouce et les doigts longs comprenant chacun deux phalanges mobiles articulées les unes par rapport aux autres et par rapport à la base de la main,
- des languettes de protection côté dorsal et côté palmaire,
- des butées aux interphalanges qui déterminent l'ouverture ou la fermeture maximum des doigts longs,
- une électronique de programmation des moteurs,
- deux fléaux, un pour l'extension et un pour la fléxion des doigts longs munis de passants et d'anneaux d'ancrage pour le tirage en fléxion et en extension,
- un moyen d'actionnement des doigts longs forme par une cage fixée à la base de la main, comprenant un moteur et un axe de rotation muni d'une roue hélicoïdale solidaire de cet axe, deux poulies munies d'axes internes sont fixées de chaque côté de cette roue hélicoïdale, sur ces axes internes son fixés les câbles de tirage et de l'autre côté aux anneaux d'ancrage des fléaux pour l'extension et la fléxion les doigts longs, ces fléaux sont également munis de passants dans lesquels chaques câbles de fléxion et d'extension s'étendent sensiblement en forme de U sur chaque paire de doigts et permettent ainsi une répartition équilibrée des forces en traction et en extension sur chaque doigt long par le coulissement de ces câbles de tirage,
- un pouce électrique composé d'une cage comprenant à son intérieur un moteur muni à son axe d'une vis sans fin qui forme avec la roue hélicoïdale un dispositif auto bloccant quand le moteur est à l'arrêt, un méplat est réalisé pour empêcher la movement de cette roue hélicoïdale,
- une bride métallique munie de deux languettes percée de deux trous et un axe servant de support à la roue hélicoïdale et à l'axe de rotation du pouce et deux ergots qui permettent de limiter les déplacements latéraux du pouce autour de son axe formé d'une vis qui est fixée sur son support positionné à 45 degrés par rapport à la base de la main, entre son support et le pouce est positionnée une rondelle en téflon qui permet le frottement et le blocage du pouce.

2. Prothèse de main à fonctionnement électrique selon la revendication 1, **caractérisée par** une cage comprenant un dispositif pour la fléxion et l'extension des quatre doigts longs formé d'un seul axe de rotation solidaire de la roue hélicoïdale et des deux poulies de tirage en fléxion ou en extension des doigts longs, chaque poulie est munie d'un axe de tirage interne et chaque cable de tirage en fléxion ou en extension est fixé, d'un côté à l'anneau d'ancrage du fléau et de l'autre côté à l'axe de la poulie correspondante.

3. Prothèse de main à fonctionnement électrique selon la revendication 1, **caractérisée par** un dispositif comprenant deux fleaux, chacun muni de quatre passants et un anneau d'ancrage servant de tirage en fléxion en extension, les quatre passants servant a l'équilibrage par paires de doigts longs des forces exercées soit en fléxion soit en extension par les cables legèrement en forme de U.

4. Prothèse de main à fonctionnement électrique selon la revendication 1 et 3 **caractérisée par** un dispositif de tirage en flèxion et en extension comprenant une cage qui sert de support à un moteur qui est muni sur son axe d'une vis sans fin qui est couplée à une vis hélicoïdale, cette vis hélicoïdale à un axe de rotation sur lequel sont fixées deux poulies munies chacune d'un axe de tirage ainsi quand la roue hélicoïdale tourne soit dans un sens soit dans l'autre, elle permet aux poulies de touner dans les deux sens et ainsi de mouvoir soit en fléxion soit en extension les doigts longs par l'intermédiaire des cables fixés sur les axes de tirage des poulies.

5. Prothèse de main à fonctionnement électrique selon la revendication 1 **caractérisée par** un pouce forme de son support et d'une cage comprenant à son intérieur un moteur muni à son axe d'une vis sans fin couplée à une roue hélicoïdale, celle-ci à un méplat et est fixé sur la bride métallique qui est munie de deux languettes percées qui servent de support à l'axe de la roue hélicoïdale, l'ensemble vis sans fin et roue hélicoïdale forment un dispositif de blocage irréversible quand le moteur ne fonctionne pas.

6. Prothèse de main à fonctionnement électrique selon la revendication 1, **caractérisé par** le support du pouce qui a une inclinaison de 45 degrés par rapport à la base de la main et sa bride métallique munie de deux ergots limitant les déplacements latéraux du pouce, sur ce support est fixée la bride métallique qui est en rotation sur son axe de fixation, une rondelle en nylon en facilite la rotation et permet au pouce de pouvoir pivoter latéralement quand on y exerce une poussée manuelle sur celui-ci.

7. Prothèse de main à fonctionnement électrique selon la revendication 1, **caractérisée par** des languettes de protection situées du côté dorsal et palmaire de la main prothétique et qui sont d'une hauteur nécessaire et suffisante pour permettre aux fléaux de coulisser et de protéger le moteur et l'électronique.

8. Prothèse de main à fonctionnement électrique selon la revendication 1 **caractérisée en ce que** les doigts ne sont pas parallèles entre eux et chaque interphalage de doigts longs est pourvue de butées limitant les extension et les fléxions maximum des doigts.

9. Prothèse de main à fonctionnement électrique selon la revendication 1 **caractérisée par** une électronique interne qui alimente les moteurs de la main prothétique, cette main est munie d'un bouton poussoir qui effleure le dorsal de la main, inserre sur son support et permet de choisir son programme d'utilisation sans que celui-ci soit visible de l'extérieur du gant de recouvrement.
